# FASCICULE DE BREVET EUROPEEN

(11) **EP 1 105 177 B1**
(45) Date de publication et mention de la délivrance du brevet: **29.06.2005**
(21) Numéro de dépôt: 99936225.4
(22) Date de dépôt: 15.08.1999
(51) Int. Cl.: A61M 16/00

(54) **DISPOSITIF POUR FOURNIR DE L'AIR MODIFIE**
VORRICHTUNG ZUR VERSORGUNG MIT MODIFIZIERTER LUFT
DEVICE FOR SUPPLYING MODIFIED AIR

(30) Priorité: 17.08.1998 CH 168498
(43) Date de publication de la demande: 13.06.2001
(73) Titulaire: MENUT, Jean-Baptiste, CH-1219 Châtelaine-Genève (CH)
(72) Inventeur: MENUT, Jean-Baptiste, CH-1219 Châtelaine-Genève (CH)
(74) Mandataire: Faltas Mikhail, William
(86) Numéro de dépôt international: PCT/CH1999/000377
(87) Numéro de publication internationale: WO 2000/010632

(56) Documents cités:
- EP-A- 0 390 684
- EP-A- 0 744 184
- WO-A-96/17641
- WO-A-97/10018
- FR-A- 2 366 833

## Description

### Domaine de l'invention

L'invention a pour objet un dispositif permettant le prélèvement d'air modifié destiné à la respiration au moyen d'un récipient à volume variable rempli d'air modifié à pression constante par l'intermédiaire d'une vanne commandée par un commutateur associé à la paroi dudit récipient.

L'expression "air modifié" utilisée ici en rapport avec la présente invention signifie de l'air dont la composition est modifiée par rapport à l'air ambiant, notamment de l'air appauvri en oxygène destiné à simuler la respiration en haute altitude.

### Etat de la technique

Un appareil d'entraînement et de thérapie décrit dans WO9637176 délivre de l'air modifié appauvri en oxygène au moyen d'un séparateur à membrane pour extraire l'oxygène de l'air ambiant. Il est par ailleurs mentionné que la teneur en oxygène peut être réduite par adsorption ou bien en ajoutant un gaz pauvre en oxygène, tel que l'azote ou de l'air expiré par l'utilisateur.

Un récipient à volume variable dans un système de respiration/d'anesthésie décrit dans EP 0 744 184 A1 est rempli d'air modifié enrichi en oxygène à pression constante par l'intermédiaire d'une vanne d'entrée commandée par deux commutateurs de fin de course associés à une partie élastique dudit récipient qui maintient l'air modifié à une pression constante dans ledit récipient. De l'air modifié à pression constante est prélevé dudit récipient et délivré à un patient par l'intermédiaire d'une vanne de sortie et d'un circuit de respiration. Lesdites vannes d'entrée et de sortie commandent respectivement l'alimentation dudit récipient en air modifie d'une part et le prélèvement d'air modifié à pression constante d'autre part. Le système décrit ici permet essentiellement de délivrer des quantités restreintes d'air modifié à débit constant. Ledit récipient à débit variable est ainsi conçu spécialement pour qu'il applique une force élastique constante afin de pouvoir délivrer l'air modifie à une pression positive constante par l'intermédiaire de ladite vanne de sortie.

Divers moyens connus pour fournir de l'air modifié destiné à la respiration sont généralement agencés de manière qu'ils délivrent de l'air modifié avec un gradient de pression positif et un débit constant, mais il ne permettent cependant que des variations restreintes du débit ainsi que de la composition de l'air modifié.

Des entraînements sportifs intensifs permettant de simuler les conditions en haute altitude nécessitent par exemple la respiration de quantités très importantes d'air appauvri en oxygène et des variations considérables du débit et de la composition dans de l'air requis pour la respiration.

Toutefois, de tels moyens connus sont généralement destinés à des applications médicales et ne conviennent pas à des applications qui exigent la respiration de quantités importantes d'air modifié et des variations du débit et de la composition de l'air modifié.

Des entraînements sportifs intensifs permettant de simuler les conditions en haute altitude nécessitent par exemple la respiration de quantités très importantes d'air appauvri en oxygène et des variations considérables du débit et de la composition dans de l'air requis pour la respiration.

### Résumé de l'invention

La présente invention est destinée à obtenir toute quantité d'air ayant toute composition modifiée requise pour la respiration et d'assurer notamment la respiration d'air modifié à des débits variables dans une gamme très large.

A cette fin, le dispositif selon l'invention présente les caractéristiques définies dans les revendications.

La présente invention prévoit un agencement particulier d'un sac compliant rempli d'air modifié à pression ambiante de manière qu'il constitue un tampon qui assure continuellement une interaction entre d'une part la diminution de son volume jusqu'à une limite inférieure prédéterminée et d'autre part le remplissage du sac compliant lorsqu'il atteint ladite limite inférieure.

L'agencement du sac compliant conformément à l'invention assure essentiellement l'aspiration de toute quantité d'air modifié requise pour la respiration par le remplissage rapide de ce sac compliant d'air modifié à pression ambiante en fonction de toute quantité d'air modifié aspirée.

Ledit sac compliant est capable de subir des variations rapides de son volume à pression ambiante, sans déformation élastique. Par conséquent, ce sac compliant est capable de se maintenir spontanément en équilibre à la pression ambiante, de varier son volume sans aucune contrainte et de se remplir très rapidement à pression ambiante, sa paroi étant soumise uniquement à la pression ambiante, de sorte que son volume peut augmenter librement en supprimant spontanément toute variation de pression.

Il est donc possible de remplir le sac compliant à pression ambiante très rapidement en réduisant son volume au strict minimum nécessaire pour obtenir tout débit d'air requis pour la respiration.

Le temps de remplissage du sac compliant peut ainsi être réduit au strict minimum nécessaire pour remplir librement le petit volume du sac compliant en quelques secondes, étant donné que le sac compliant à pression ambiante ne présente aucun obstacle à l'entrée de l'air modifié.

L'aspiration de toute quantité d'air modifié à pression ambiante requise pour la respiration est assurée au moyen d'un tuyau d'aspiration permettant de produire toute dépression requise pour commander exactement le débit de l'air aspiré ainsi que la durée d'aspiration. La perte de charge dans le tuyau d'aspiration sera par ailleurs négligeable même à des débits d'aspiration très élevées, la valeur de cette perte étant inférieure à 40 mm de colonne d'eau à un débit d'aspiration extrêmement élevé de 600 litre/minute dans un tuyau d'un diamètre de 38 mm.

La cadence du remplissage répété du sac compliant à pression ambiante est ainsi adaptée continuellement et commandée exactement en fonction de l'aspiration de toute quantité d'air modifié aspirée requise pour la respiration.

Le sac compliant peut par conséquent présenter un volume très petit de quelques litres et être rempli très rapidement en quelques secondes, de sorte que son remplissage répété plusieurs fois par minute permet de délivrer constamment des débits d'air très importants par rapport à son volume restreint.

Le remplissage répété du sac compliant à pression ambiante peut par ailleurs être commandé exactement en même temps que l'aspiration d'air modifié à tout débit requis.

Des essais ont démontré qu'un sac compliant agencé conformément à l'invention permet de varier le débit de l'air modifié aspiré du sac compliant dans une gamme très large allant de 0 jusqu'à 200 l/min., ou même beaucoup plus.

Le sac compliant peut être formé avantageusement d'une feuille en matière plastique souple et extrêmement mince d'une épaisseur de quelques centièmes de millimètre, de sorte qu'il soit apte à subir des changements de volume rapides sans déformation élastique ou modification de sa superficie.

De l'air ayant toute composition requise pour la respiration s'obtient avantageusement au moyen d'un éjecteur couplé au sac compliant comme il est décrit par la suite.

Le remplissage du sac compliant est réalisé avantageusement au moyen d'un éjecteur et remplit les conditions suivantes conformément à l'invention:
1. L'alimentation du sac compliant en air modifié délivré par l'éjecteur est égale à la demande d'air prélevé du sac compliant.
2. Le fonctionnement intermittent de l'éjecteur est commandé par l'intermédiaire du sac compliant en fonction du prélèvement d'air du sac compliant.
3. Le sac compliant et l'éjecteur sont agencés dans une boucle de rétroaction afin de commander le remplissage répété du sac compliant uniquement au moyen d'un commutateur associé au sac compliant et d'une vanne associée à l'éjecteur.
4. La sortie de l'éjecteur débouche librement dans le sac compliant à pression ambiante.
5. La pression et le débit du gaz injecté sont maintenus constants dans l'éjecteur.
6. Le débit d'air aspiré dépend de la section d'entrée d'air dans l'éjecteur.
7 La composition et le débit de l'air modifié délivré par l'éjecteur dépendent essentiellement de la section d'entrée d'air aspiré dans l'éjecteur.
8. La combinaison d'un sac compliant muni d'un commutateur avec un éjecteur muni d'une vanne permet ainsi de fournir toute quantité d'air modifié ayant toute composition requise.

### Description détaillée

L'invention peut être illustrée à l'aide des formes d'exécution d'un dispositif comprenant un sac compliant et un injecteur associé à une source d'azote et destiné à fournir de l'air appauvri en oxygène.
Fig. 1 montre schématiquement une forme d'exécution d'un dispositif comprenant un sac compliant et un éjecteur associé à une source d'azote et destiné à fournir de l'air appauvri en oxygène.
Fig. 2 montre une coupe schématique de l'éjecteur du dispositif selon Fig. 1.
Fig. 3 illustre la variation de la fraction d'oxygène de l'air en fonction du rapport de l'air ambiant aspiré à l'azote injecté dans l'éjecteur selon Fig. 2.
Fig. 4a à Fig. 4d montrent respectivement la variation périodique du volume du sac compilant selon Fig. 1 pendant le prélèvement de 5, 25, 100 et 200 l/min d'air en même temps que le remplissage répété de ce sac compliant.
Fig. 5 montre schématiquement une variante du dispositif selon Fig. 1.

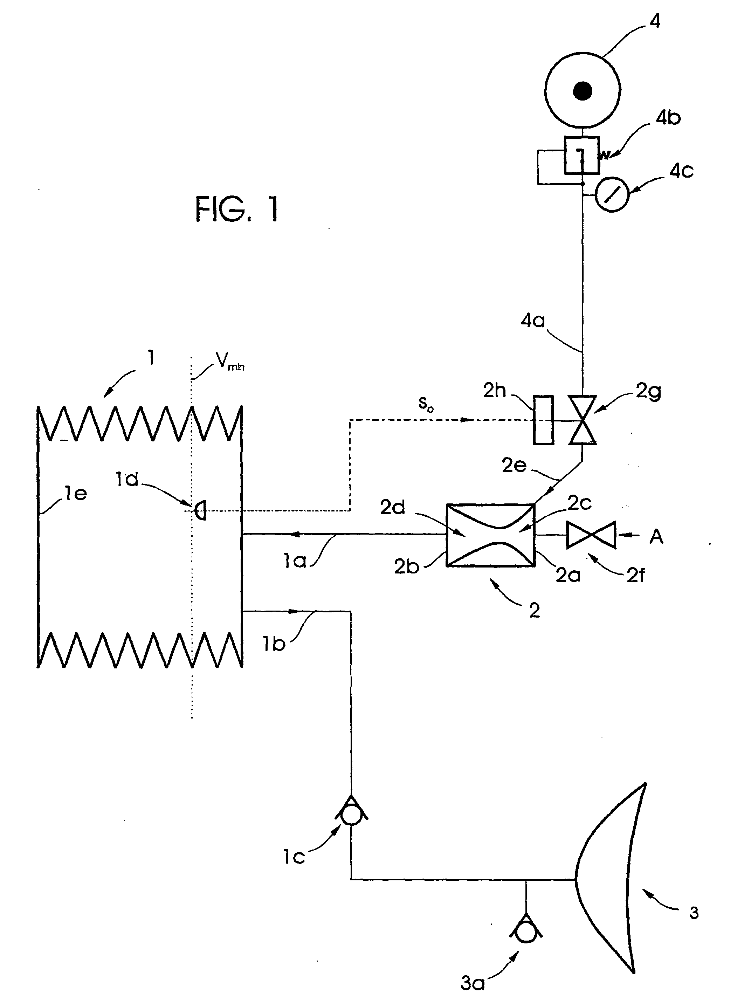

Le dispositif selon Fig. 1 comprend un sac compilant 1 communiquant avec la sortie d'un éjecteur 2 par un tuyau d'amenée 1a et avec un tuyau d'aspiration 1b associé à un masque respiratoire 3 et à deux soupapes de non-retour 1c et 3a afin d'assurer l'aspiration à travers la soupape 1c et l'expiration à travers la soupape 3a.

Le sac 1 est choisi de manière qu'il soit parfaitement compilant et capable de subir des changements de volume à pression constante, afin qu'il soit capable de se remplir d'air à pression ambiante conformément à l'invention.

Le sac compliant 1 contient un commutateur représenté par un contact 1d disposé dans un plan indiqué par la ligne en traits mixtes Vₘᵢₙ qui correspond à une limite inférieure prédéterminée du volume du sac 1. Le sac compilant 1 est muni d'une surface conductrice interne le servant à établir un contact électrique avec le commutateur 1d lorsque le volume du sac compliant 1 atteint sa limite inférieure Vₘᵢₙ selon Fig. 1

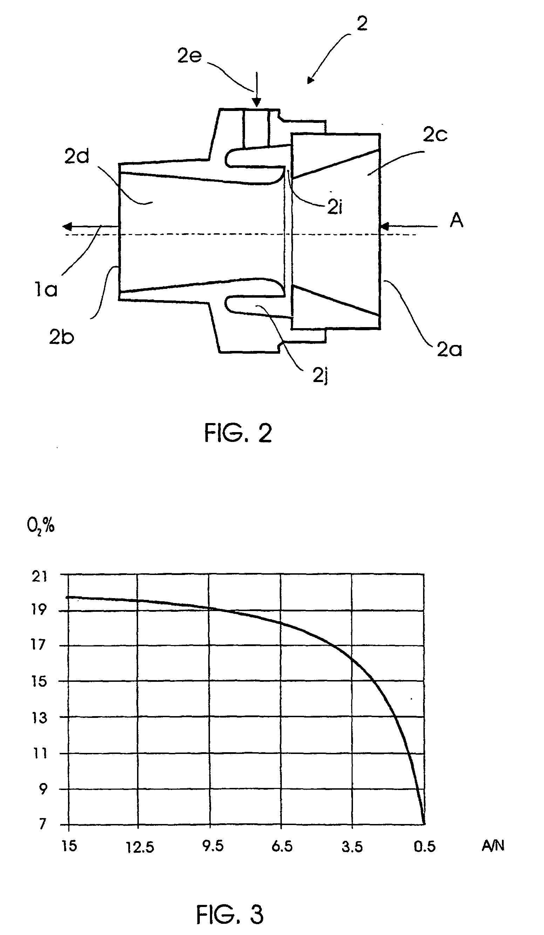

L'éjecteur 2 selon Fig. 1 comporte un canal d'écoulement longitudinal formé d'un canal d'entrée convergeant 2c communiquant avec une extrémité d'entrée 2a et avec un canal de sortie divergeant 2d communiquant avec une extrémité de sortie 2b de l'éjecteur 2. Le canal d'entrée convergeant 2c communique avec l'atmosphère par l'intermédiaire d'une vanne d'air 2f et avec une source d'azote sous pression 4, sous forme d'une bouteille d'azote en l'occurrence, par l'intermédiaire d'un tuyau d'amenée en azote 4a, d'une électrovanne 2g et d'un tuyau 2e d'amenée d'azote sous pression, d'une fente d'injection annulaire 2i et d'un compartiment d'entrée annulaire 2j agencés dans l'éjecteur 2 (voir Fig. 2). Le canal de sortie divergeant 2d communique à l'extrémité de sortie opposée 2b de l'éjecteur 2 avec le sac compliant 1 par l'intermédiaire du tuyau la d'amenée.

La vanne d'air 2f est prévue à proximité de l'extrémité d'entrée 2a de l'éjecteur afin d'ajuster la quantité d'air ambiant aspiré dans l'éjecteur 1, indiqué par la lettre A (Fig. 1 et 2).

Le tuyau d'amenée d'azote 4a est par ailleurs muni d'un détendeur 4b et d'un manomètre 4c permettant de contrôler la pression de l'azote fourni à l'électrovanne 2g.

Fig. 1 montre en outre que le commutateur 1d est relié à l'électrovanne 2g par l'intermédiaire d'un temporisateur 2h, comme il est indiqué par la ligne en traits mixtes So.

Le sac compliant 1 est associé au tuyau 2e d'amenée d'azote à l'éjecteur 2 par l'intermédiaire du commutateur 1d et de l'électrovanne 2g de manière à commander l'ouverture et la fermeture de l'électrovanne 2g par l'intermédiaire d'un temporisateur 2h en fonction du volume du sac compliant 1.

L'électrovanne 2g est une vanne électromagnétique associée au commutateur 1d et au temporisateur 2h et est agencée de manière qu'elle soit normalement fermée dans sa position de repos par un ressort, qu'elle soit activée et amenée dans sa position active d'ouverture aussitôt que le volume du sac compliant atteint sa limite inférieure Vₘᵢₙ et le commutateur 1d établit un contact électrique avec la surface interne le du sac, qu'elle soit maintenue ouverte dans sa position active à l'aide du temporisateur 2h pendant une période prédéterminée permettant de remplir le sac compliant 1 d'air à pression ambiante, et qu'elle soit ensuite refermée et ramenée dans sa position de repos.

L'amenée d'azote sous pression à l'éjecteur 2 par l'électrovanne 2a est ainsi commandée conformément à l'invention par l'intermédiaire du sac compliant 1 associé au commutateur 1d.

Fig. 1 représente le tuyau d'amenée d'azote 2c par une ligne inclinée afin d'indiquer que l'éjecteur 2 est prévu en l'occurrence pour effectuer l'injection latérale ainsi que l'écoulement longitudinal dans les canaux 2c et 2d ainsi que l'aspiration axiale d'air ambiant.

On a pu assurer le fonctionnement satisfaisant du dispositif selon Fig. 1 au moyen d'un sac compliant 1 formé d'une feuille fine de Mylar® munie d'une surface interne métallisée le destinée à établir un contact électrique avec le commutateur 1d.

Des essais ont été effectués dans le cadre de la présente invention avec un dispositif tel que décrit selon Fig. 1 comprenant un sac 1 en Mylar métallisé en combinaison avec un éjecteur 2 de type connu, obtenu sous le nom de "Airmover" du type AM40. Ces essais ont démontré que le dispositif décrit selon Fig. 1 permet de faire varier la composition ainsi que le débit de l'air prélevé du sac compliant dans des gammes très larges.

Fig. 2 montre plus en détail une coupe schématique d'un éjecteur du type décrit selon Fig. 1, les mêmes éléments portant les mêmes références dans ces deux figures.

L'éjecteur selon Fig. 2 comprend un canal d'écoulement longitudinal composé d'un canal d'entrée convergeant 2c débouchant dans un canal de sortie divergent 2d et d'un tuyau latéral 2e d'amenée d'azote sous pression. Comme il ressort de Fig. 2, le tuyau latéral d'amenée d'azote 2e communique avec le canal d'écoulement longitudinal de l'éjecteur par un compartiment annulaire 2j d'amenée d'azote relié à une fente annulaire 2i qui débouche dans le canal d'entrée convergent 2c de manière à provoquer un écoulement longitudinal. La configuration de la fente annulaire 2i est conçue spécialement de manière à diriger un jet le long de la paroi du canal d'écoulement, à entraîner de l'air ambiant aspiré à l'extrémité d'entrée 2a de l'éjecteur, à mélanger l'air ambiant à l'azote dans le canal divergeant et à refouler de l'air appauvri en oxygène par l'extrémité de sortie 2b.

Le brevet CH 595 558 contient une explication détaillée de la structure et du mode de fonctionnement d'un éjecteur du même type que l'éjecteur 2 selon Fig. 1 et 2. Un éjecteur du type selon Fig. 2 permet notamment de délivrer des débits élevés d'air modifié et le remplissage rapide du sac compilant à pression ambiante conformément à la présente invention.

Fig. 3 montre la variation du pourcentage d'oxygène de l'air en fonction du rapport A/N de la quantité d'air A aspiré à la quantité d'azote N injecté dans ledit type d'éjecteur.

Comme il ressort de Fig. 3, ledit type d'éjecteur permet de faire varier le rapport air/azote dans l'air modifié fourni dans un grande gamme s'étendant de A/N = 0,5 jusqu'à 15 et la teneur en oxygène dans une grande gamme s'étendant de O₂ = 7 % jusqu'à environ 19 %, ce qui correspond, au niveau de la mer, à pression normale de 760 mm Hg, à des pressions partielles d'oxygène de 53 mm Hg et de 145 mm Hg, soit des pressions partielles d'oxygène que l'air présente dans la nature à des altitudes de 8400 m et 800 m respectivement.

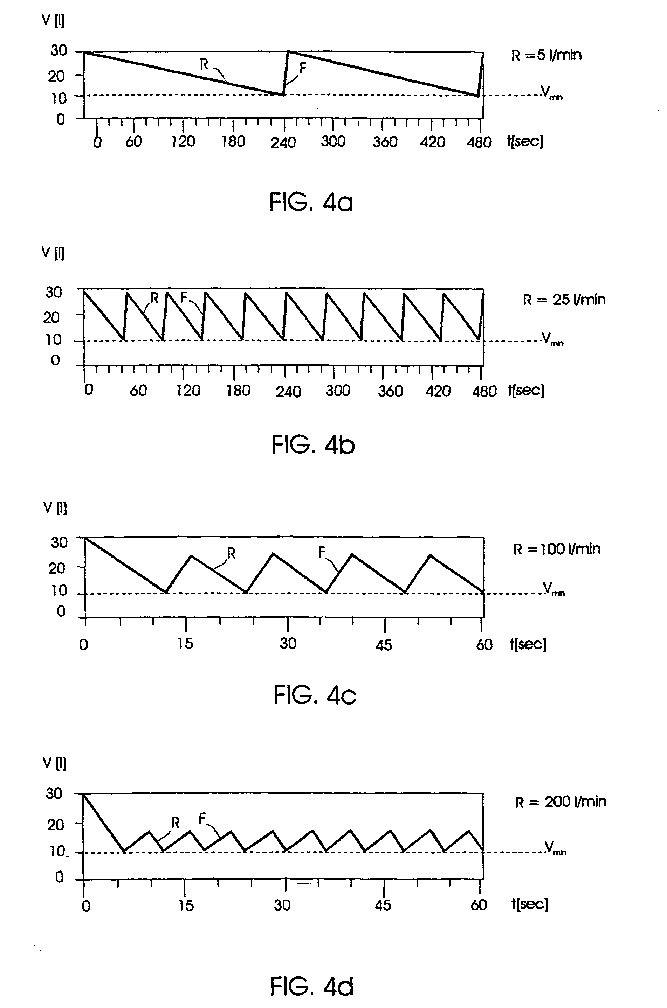

Fig. 4a à 4d illustrent le fonctionnement du dispositif décrit selon Fig. 1 par des lignes en dents de scie représentant les changements de volume successifs du sac compilant 1 en fonction du temps au cours du prélèvement de différents débits d'air et du fonctionnement intermittent de l'éjecteur.

Fig. 4a illustre la variation du volume du sac 1 pendant le prélèvement de 5 l/min d'air et des phases de remplissage d'une durée de 4 secondes chacune. La consommation d'air d'un individu au repos correspond à 5 l/min.

Fig. 4a montre les cycles de remplissage et d'évacuation comprenant chacun d'une part une phase de remplissage durant 4 sec, représentée par une branche ascendante oblique F qui correspond à l'expansion du sac d'une limite inférieure Vₘᵢₙ de 101 jusqu'à une limite supérieure d'environ 301 pendant le fonctionnement de l'éjecteur en même temps que le prélèvement et l'évacuation d'air modifié pour la respiration au moyen du tuyau d'aspiration 1b. Chacun desdits cycles comprend d'autre part une phase d'évacuation représentée par une branche descendante oblique R qui correspond à la diminution de volume du sac jusqu'à la limite inférieure de 10 l.

Chaque phase de remplissage F est provoquée par le fonctionnement de l'éjecteur 2 aussitôt que le volume du sac 1 atteint la limite inférieure Vₘᵢₙ de 10 l. Le commutateur 1d établit un contact électrique avec la surface conductrice interne le du sac 1 afin de provoquer l'ouverture de l'électrovanne 2g, d'injecter de l'azote et d'aspirer de l'air ambiant dans l'éjecteur 2, de remplir ainsi le sac 1 d'air modifié à pression ambiante. L'électrovanne 2g est dans ce cas maintenue dans sa position ouverte à l'aide du temporisateur 2h pendant 4 secondes afin de remplir le sac 1 et augmenter son volume de 10 l à environ 30 l. L'électrovanne 2g est alors ramenée automatiquement par un ressort de rappel et se referme dans sa position de repos pendant la phase d'évacuation R correspondant à la diminution du volume du sac jusqu'à la limite inférieure de 10 l.

Il convient de noter que le prélèvement d'air pour la respiration s'effectue dans ce cas avec un débit moyen de 5 1 pendant l'ensemble des cycles de remplissage et d'évacuation, c'est-à-dire aussi bien pendant les phases de remplissage F lors du fonctionnement de l'éjecteur 2 que pendant les phases d'évacuation R.

La description de Fig. 4a s'applique essentiellement aux Fig. 4b, 4c et 4d et les différences principales seront mentionnées ci-dessous.

Fig. 4b représente la variation du volume du sac compilant 1 au cours du prélèvement de 25 l/min d'air, ce débit étant augmenté cinq fois par rapport à Fig. 4a et la fréquence des cycles F/R ainsi que les pentes des lignes en dents de scie selon Fig. 4b étant augmentées en conséquence.

Fig. 4c représente la variation du volume du sac compilant 1 au cours du prélèvement de 100 l/min d'air, ce débit étant multiplié 20 fois par rapport à Fig. 4a et la fréquence des cycles F/R ainsi que les pentes des lignes en dents de scie selon Fig. 4c étant augmentées en conséquence.

Fig. 4d représente la variation du volume du sac compilant 1 au cours du prélèvement de 200 l/min d'air, ce qui correspond à la consommation d'air d'un sportif de très haut niveau en plein effort, ce débit de prélèvement d'air étant multiplié 40 fois par rapport à la Fig. 4a et la fréquence des cycles F/R ainsi que les pentes des lignes en dents de scie selon Fig. 4d étant augmentées en conséquence.

Fig 4a à 4d montrent que le sac compilant d'un volume de remplissage utile de seulement 20 litres peut délivrer constamment de 5 l/min à 200 l/min d'air. Le débit d'air délivré par le sac compliant dépend de la fréquence et de la vitesse de son remplissage plutôt que de son volume, ce débit pouvant par ailleurs être augmenté davantage.

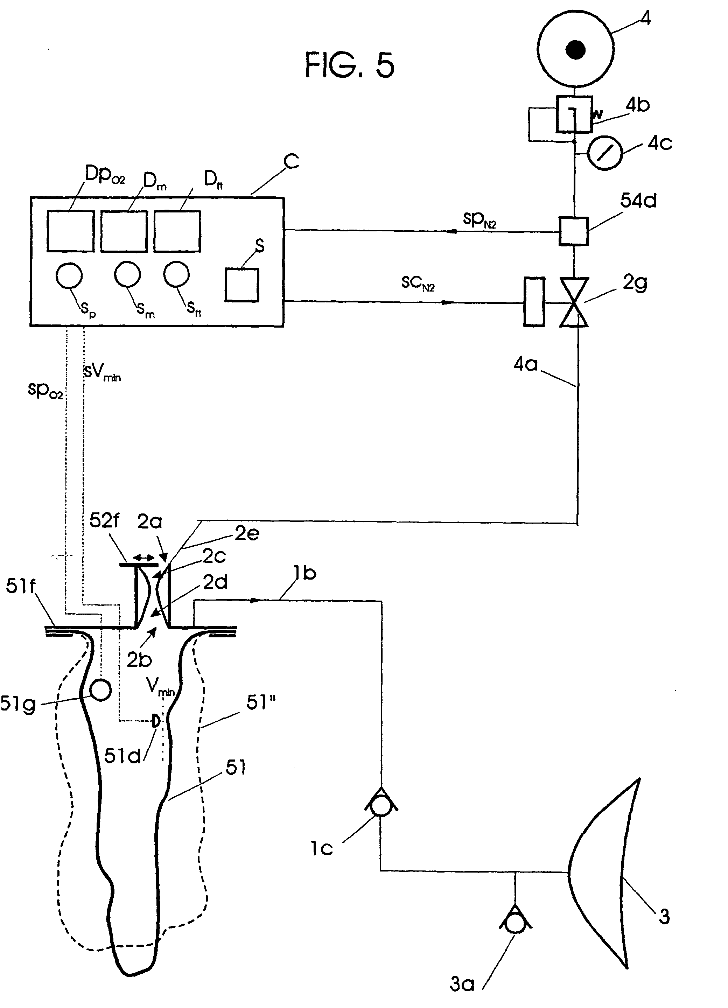

Fig. 5 représente schématiquement une variante du dispositif décrit selon Fig. 1, les éléments identiques étant désignés par les mêmes références dans ces deux figures.

La variante selon Fig. 5 comprend un sac compliant 51 qui est suspendu à une paroi horizontale fixe 51 f et présente une ouverture supérieure obturée de façon étanche par cette paroi.

Le sac compliant 51 communique avec la sortie d'un éjecteur 2 par l'intermédiaire d'un tuyau d'amenée la qui traverse la paroi horizontale 51f de façon étanche. L'éjecteur 2 selon cette variante est essentiellement le même que l'éjecteur déjà décrit selon Fig. 1 et 2. Le sac compliant 51 communique également avec un masque respiratoire 3 par l'intermédiaire d'un tuyau d'aspiration 1b qui traverse la paroi horizontale 51f de façon étanche et comporte une soupape de non retour 1c. Le masque 3 est également muni d'une soupape de non retour 3c pour l'évacuation de l'air expiré.

Un commutateur de proximité 51d est monté fixe à l'intérieur du sac compliant 51 à un endroit indiqué par une ligne en traits mixtes qui correspond à une limite inférieure prédéterminée Vₘᵢₙ du volume du sac 51. Ce commutateur de proximité 51d délivre un signal de détection sVₘᵢₙ lorsque le volume du sac compliant atteint sa limite inférieure Vₘᵢₙ. Fig. 5 représente ce signal de détection par la ligne en points tirets marquée sVₘᵢₙ.

Le sac compliant 51 contient en outre une sonde à oxygène 51g qui délivre un signal de pression partielle d'oxygène sp_{O2} correspondant à la pression partielle de l'oxygène dans l'air contenu dans le sac 51. Fig. 5 représente ce signal de pression partielle d'oxygène par la ligne en point tirets marquée sp_{O2}.

Un conduit d'amenée d'azote 4a relie la source d'azote sous pression 4 à l'électrovanne 2g par l'intermédiaire d'un détendeur 4b, d'un manomètre 4c et d'un capteur de pression 54d qui émet un signal sp_{N2} correspondant à la pression d'azote dans la conduite d'amenée 4a. Fig. 5 représente ce signal de pression d'azote par une ligne en points tirets marquée sp_{N2}.

Le dispositif selon Fig. 5 est muni en outre d'un panneau de contrôle C qui est associé à un circuit électronique non représenté, qui reçoit le signal de détection sVₘᵢₙ du commutateur de proximité 51d, le signal de pression d'oxygène sp_{O2} de la sonde à oxygène 51g et le signal de pression d'azote sp_{N2} du capteur de pression 54d, et qui transmet un signal de commande sc_{N2} à l'électrovanne 2g afin de remplir le sac compilant 51 chaque fois que son volume atteint sa limite inférieure Vₘᵢₙ.

Le panneau de contrôle C comprend un écran à trois zones d'affichage Dp_{O2}, Dₘ, D_{ft}, un bouton de commande S associé à un interrupteur principal non représenté (du type "ON/OFF") pour la mise en service et l'arrêt du dispositif selon Fig. 5 et trois boutons de sélection Sₚ, Sₘ, S_{ft} servant respectivement à choisir l'affichage de la pression partielle d'oxygène p_{O2}, exprimée en mm Hg dans la zone d'affichage Dp_{O2}, ou bien l'affichage de altitude correspondant à p_{O2} exprimée soit en mètres dans la zone d'affichage Dₘ soit en pieds dans la zone d'affichage D_{ft}.

Lorsque le volume du sac compliant 51 atteint sa limite inférieure Vₘᵢₙ, le signal de détection sVₘᵢₙ est émis par le commutateur de proximité 51d et transmis au circuit électronique associé au panneau de contrôle C. Ledit circuit électronique transmet alors un signal de commande sc_{N2} à électrovanne 2g afin de provoquer son ouverture, de relier la source d'azote 4 à l'éjecteur 2, d'injecter de l'azote sous pression et d'aspirer de l'air ambiant dans l'éjecteur et de remplir le sac compilant 51 d'un mélange d'azote et d'air ambiant ayant la composition requise.

Le circuit électronique associé au panneau de contrôle comprend un temporisateur non représenté qui maintient le signal de commande sc_{N2} pendant un temps suffisant pour remplir le sac compliant 51. L'électrovanne 2g se referme automatiquement sous l'action d'un ressort de rappel lorsque le signal sc_{N2} disparaît afin de terminer le remplissage du sac compilant 51.

Ledit circuit électronique associé au panneau de contrôle C est agencé de manière qu'il reçoit constamment le signal de pression d'azote sp_{N2} du capteur de pression 54d lorsque le dispositif décrit est mis en service par le commutateur principal S et qu'il délivre un signal d'avertissement sonore lorsque la pression d'azote diminue à une limite inférieure requise pour le fonctionnement de l'éjecteur, à savoir lorsque la bouteille d'azote 4 est vide.

La mise en oeuvre de l'invention au moyen d'un dispositif tel que décrit selon Fig. 1 permet de combiner les avantages suivants:
1. Toute quantité d'air requise pour la respiration est fournie par le sac compilant qui est rempli chaque fois que son volume atteint sa limite inférieure.
2. Le prélèvement d'air du sac compliant commande constamment son remplissage répété en fonction de la quantité d'air prélevée dans chaque cas.
3. Le remplissage répété du sac compliant est commandé par la variation de son volume, est adapté constamment à toute quantité d'air consommée et peut être assuré par une vanne associée à une source de gaz sous pression et commandée par un commutateur associé au sac compliant.
4. Le volume du sac compliant peut être réduit au strict minimum nécessaire afin que son remplissage répété permette le prélèvement de toute quantité d'air requise.
5. Le débit d'air prélevé peut être augmenté de 0 à au moins 200 l'min.
6. Le sac compliant constitue un tampon de sortie qui élimine le stockage dans un réservoir encombrant, le volume du sac compliant étant négligeable par rapport au débit d'air prélevé pendant de longues périodes.
7. L'emploi d'un éjecteur pour la mise en oeuvre de l'invention permet de mélanger intimement toute quantité d'un gaz injecté sous pression et d'air aspiré dans l'éjecteur.
8. Un éjecteur de faible taille constitue un moyen particulièrement simple et fiable permettant de délivrer instantanément l'air nécessaire pour le remplissage répété du sac compliant.
9. Le sac compliant reste à pression constante et une vanne disposée à l'entrée d'air de l'éjecteur permet de faire varier dans une très grande gamme la composition de l'air délivré par l'éjecteur.
10. La combinaison d'un éjecteur communiquant directement avec un sac compliant conformément à l'invention permet ainsi de fournir constamment toute quantité d'air ayant la composition requise.
11. Possibilité de faire varier considérablement la composition ainsi que le débit de l'air délivré conformément à l'invention.
12. L'invention est mise en oeuvre ainsi au moyen d'une combinaison particulièrement simple d'éléments très fiables dont le nombre et la taille sont réduits au strict minimum nécessaire pour délivrer toute quantité d'air ayant la composition requise et permettant la respiration prolongée.

Le sac compilant utilisé pour la mise en oeuvre de l'invention peut avoir toute forme appropriée et ses dimensions peuvent être réduites et adaptées à toute quantité d'air requise pour la respiration.

Diverses variantes et modifications peuvent être envisagées dans le cadre de la présente invention par rapport aux dispositifs décrits à titre d'exemple.

Au lieu de commander le remplissage répété du sac compliant au moyen d'un commutateur avec un temporisateur, on pourrait également utiliser deux commutateurs, notamment des commutateurs de proximité, agencés respectivement à une limite inférieure et à une limite supérieure du volume du sac compliant de manière à commander l'ouverture ainsi que la fermeture de l'électrovanne au début et à la fin de chaque période de remplissage.

On peut également utiliser avantageusement des moyens similaires comprenant une source d'oxygène sous pression pour délivrer de l'air enrichi d'oxygène.

Divers essais d'entraînement intensifs effectués par des athlètes de haut niveau en respirant de l'air appauvri en oxygène, fourni conformément à l'invention au moyen d'un dispositif tel que décrit, ont démontré un fonctionnement satisfaisant de ce dispositif et ont fourni des résultats prometteurs, notamment une augmentation appréciable de la consommation maximale d'oxygène.

## Revendications

1. Dispositif permettant le prélèvement d'air modifié destiné à la respiration et comportant un récipient à volume variable relié à une source d'air modifié par l'intermédiaire d'une vanne commandée par un commutateur qui est associé à la paroi dudit récipient à volume variable et provoque l'ouverture de ladite vanne lorsque le volume dudit récipient atteint une limite inférieure prédéterminée, **caractérisé en ce que**:
(a) un sac compliant (1) capable de subir des changements de volume rapides à pression ambiante sans déformation élastique et de se remplir d'air modifié à pression ambiante constitue ledit récipient à volume variable;
(b) ledit sac compliant (1) communique avec un tuyau d'aspiration (1b) permettant de prélever toute quantité d'air modifié à pression ambiante requise pour la respiration;
(c) ledit commutateur (1d) est associé à la paroi dudit sac compliant (1) et à ladite vanne (2g), de manière qu'il provoque l'ouverture de ladite vanne de commande (2g) chaque fois que le volume du sac compliant (1) atteint ladite limite inférieure prédéterminée (Vₘᵢₙ) et
(d) la combinaison de (a), (b) et (c) est prévue de manière que ledit sac compliant (1) soit rempli d'air modifie à pression ambiante en fonction de toute quantité d'air modifié à pression ambiante prélevée requise pour la respiration.

2. Dispositif selon la revendication 1, **caractérisé en ce que** ledit sac compliant (1) communique directement avec la sortie (2b) d'un éjecteur (2) qui comprend deux entrées (2e et 2c) reliées respectivement à une source de gaz sous pression (4) et à l'atmosphère par l'intermédiaire de ladite vanne (2) et d'une vanne (2f; 52f) d'aspiration d'air ambiant, ledit éjecteur (2) comprenant une sortie (2b) communiquant avec ledit sac compliant (1) de manière que l'ouverture de ladite vanne (2) provoque l'injection dudit gaz sous pression dans un rapport prédéterminé dans de l'air ambiant aspiré dans l'éjecteur (2) et remplit ledit sac compliant (1) d'air modifié à pression ambiante ayant toute composition requise pour la respiration.

## Patentansprüche

1. Vorrichtung zum Entnehmen von modifizierter Atemluft, wobei die Vorrichtung einen Behälter mit veränderlichem Volumen aufweist, der an eine Quelle modifizierter Luft über ein Ventil angeschlossen ist, das von einem der Behälterwand zugeordneten Schalter gesteuert wird, der das genannte Ventil jeweils öffnet, wenn das Volumen des genannten Behälters eine vorbestimmte untere Grenze erreicht, **dadurch gekennzeichnet, dass**:
(a) ein nachgiebiger Sack (1), der rasche Volumenänderungen bei Atmosphärendruck ohne elastische Deformation erfahren und mit modifizierter Luft bei Atmosphärendruck gefüllt werden kann, den genannten Behälter mit veränderlichem Volumen bildet,
(b) der genannte nachgiebige Sack (1) in Verbindung mit einem Saugrohr (1b) steht, das die Entnahme jeder Menge der zum Atmen erforderlichen modifizierten Luft bei Atmosphärendruck gestattet,
(c) der genannte Schalter (1d) der Wand des nachgiebigen Sacks (1) sowie dem genannten Ventil (2g) zugeordnet ist, so dass er das genannte Steuerventil (2g) jeweils öffnet, wenn das Volumen des nachgiebigen Sacks (1) die genannte vorbestimmte untere Grenze (Vₘᵢₙ) erreicht, und
(d) die Kombination von (a), (b) und (c) derart vorgesehen ist, dass der genannte nachgiebige Sack (1) in Abhängigkeit von jeder entnommenen, zum Atmen erforderlichen Menge modifizierter Luft bei Atmosphärendruck mit modifizierter Luft bei Atmosphärendruck geftillt wird.

2. Vorrichtung nach Anspruch 1, **dadurch gekennzeichnet, dass** der genannte nachgiebige Sack (1) unmittelbar mit dem Austritt (2b) eines Ejektors (2) mit zwei Eintritten (2e und 2c) in Verbindung steht, die über das genannte Ventil (2) mit einer Gasquelle (4) unter Druck bzw. über ein Ansaugventil (2f; 52f) mit der Atmosphäre zum Ansaugen von Umgebungsluft verbunden sind, wobei der genannte Ejektor (2) einen Austritt (2b) aufweist, der mit dem genannten nachgiebigen Sack (1) in Verbindung steht, so dass das Öffnen des genanten Ventils (2) das Injizieren von Gas unter Druck in einem vorbestimmten Verhältnis in die Umgebungsluft bewirkt, die in den Ejektor (2) angesaugt wird und den genannten nachgiebigen Sack (1) mit modifizierter Luft jeder zum Atmen erforderlichen Zusammensetzung bei Atmosphärendruck füllt.

## Claims

1. Device for drawing off modified air for respiration, said device comprising a recipient of variable volume connected to a source of modified air via a valve controlled by a switch that is associated with the wall of said recipient of variable volume and causes said valve to open when the volume of said recipient reaches a predetermined lower limit, **characterized in that**:
(a) a compliant bag (1) that is capable of undergoing rapid changes in volume at ambient pressure without elastic deformation and of being filled with modified air at ambient pressure constitutes said recipient of variable volume;
(b) said compliant bag (1) communicates with a suction tube (1b) enabling any amount of modified air at ambient pressure required for respiration to be drawn off;
(c) said switch (1d) is associated with the wall of said compliant bag (1) and with said valve (2g) so that it causes said control valve (2g) to open each time the volume of said compliant bag (1) reaches said predetermined lower limit (Vₘᵢₙ) and
(c) the combination of (a), (b) and (c) is arranged so that said compliant bag (1) is filled with modified air at ambient pressure according to any required amount of modified air at ambient pressure drawn off for respiration.

2. Device according to claim 1, **characterised in that** said compliant bag (1) communicates directly with the outlet (2b) of an ejector (2) comprising two inlets (2e and 2c) connected respectively to a source of gas under pressure (4) and to the atmosphere via said valve (2) and an intake valve (2f, 52f) for ambient air, said ejector (2) comprising an outlet (2b) communicating with said compliant bag (1) so that opening said valve (2) causes the injection of said gas under pressure in a predetermined ratio into the ambient air drawn into the ejector (2) and fills said compliant bag (1) with modified air at ambient pressure having any composition required for respiration.
